# EUROPÄISCHE PATENTSCHRIFT

(11) **EP 2 248 484 B1**
(45) Veröffentlichungstag und Bekanntmachung des Hinweises auf die Patenterteilung: **06.07.2011**
(21) Anmeldenummer: 09159534.8
(22) Anmeldetag: 06.05.2009
(51) Int. Cl.: A61B 19/00, A61F 2/46

(54) **Werkzeug zum Erfassen von Ebenen eines Knochens und zugeordnetes Datenverarbeitungsverfahren**
Tool for recording levels of a bone and allocated data processing method
Outil de détection de surfaces d'un os et procédé de traitement des données correspondant

(43) Veröffentlichungstag der Anmeldung: 10.11.2010
(73) Patentinhaber: BrainLAB AG, 85622 Feldkirchen (DE)
(72) Erfinder: Neubauer, Timo, 85586 Poing (DE); Millahn, Manuel, 80799 München (DE)
(74) Vertreter: Schwabe - Sandmair - Marx

(56) Entgegenhaltungen:
- EP-A- 1 889 584
- WO-A-2004/041097
- WO-A-2007/085085

## Beschreibung

Die vorliegende Erfindung betrifft ein Werkzeug zum Erfassen von durch insbesondere anatomische Landmarken definierten Ebenen einer Körperstruktur (z. B. Knochen), insbesondere ein Werkzeug in L-förmiger Gestalt. Weiter betrifft die vorliegende Erfindung ein Datenverarbeitungsverfahren, um mittels des Werkzeuges erfasste Daten zu verarbeiten. Die Erfindung betrifft weiter ein Programm, das das Datenverarbeitungsverfahren mittels eines Computers durchführt, sowie ein Navigationssystem mit einem derartigen Computer.

Die vorliegende Erfindung betrifft insbesondere das Gebiet der computer-gestützten Navigation in der Chirurgie (IGS oder "Image Guided Surgery"). Auf diesem Gebiet ist es notwendig, die Position der Knochen im Raum durch ein chirurgisches Navigationssystem zu erfassen. Unter einem chirurgischen Navigationssystem bzw. Navigationssystem wird ein System verstanden, das aus wenigstens einer Markervorrichtung, einem Sender, der elektromagnetische Wellen bzw. Strahlung und/oder Ultraschallwellen emittiert, und einem Empfänger, der elektromagnetische Wellen bzw. Strahlung und/oder Ultraschallwellen empfängt, sowie einer elektronischen Datenverarbeitungsvorrichtung, die an den Empfänger und/oder den Sender angeschlossen ist, besteht. Die Datenverarbeitungsvorrichtung (z. B. ein Computer) umfasst dabei einen Prozessor (CPU), einen Arbeitsspeicher, vorteilhaft eine Anzeigemöglichkeit (z. B. eine visuelle Anzeigemöglichkeit wie einen Monitor und/oder eine Audioanzeigemöglichkeit wie einen Lautsprecher) sowie vorteilhaft einen permanenten Datenspeicher. Die Datenverarbeitungsvorrichtung verarbeitet dabei Navigationsdaten, die von dem Empfänger an sie weitergegeben werden und kann vorteilhaft Hinweisinformationen über die Anzeigemöglichkeit an einen Anwender ausgeben. Die Navigationsdaten können in dem permanenten Datenspeicher abgelegt werden und beispielsweise mit Daten verglichen werden, die dort zuvor bereitgestellt worden sind. Um die Position der Knochen zu erfassen, werden beispielsweise mittels eines Pointers Landmarken am Knochen abgegriffen, um so die Position der Landmarke dem Navigationssystem mitzuteilen, um so ein dreidimensionales Bild des Knochens, das die Lage des Knochens darstellt, erstellen zu können. Kurz ausgedrückt; wird der Knochen mit Hilfe des Navigationssystems registriert. Ein Pointer ist ein Werkzeug, insbesondere ein Stab mit mehreren, vorteilhaft -drei daran befestigten Markern, wobei die relative Position zwischen den Markern und der Pointerspitze bekannt ist. Dadurch kann durch Detektion der Lage der Marker bestimmt werden, wo sich die Pointerspitze befindet. Wird die Pointerspitze mit einer Landmarke in Kontakt gebracht, ist somit die Lage der Landmarke des Knochens bekannt.

Aufgabe eines Markers ist, von einer Markererfassungseinrichtung (Detektionsvorrichtung, wie z.B. einer Kamera oder einem Ultraschallempfänger) des Navigationssystems erfasst zu werden, so dass seine Lage (d.h. Position und/oder Ausrichtung) im Raum ermittelt werden kann. Solche Marker können aktive Marker sein. Ein aktiver Marker emittiert beispielsweise elektromagnetische Strahlung bzw. Wellen, wobei diese Strahlung im infraroten, sichtbaren und/oder ultravioletten Spektralbereich liegen kann. Der Marker kann aber auch passiv sein, d.h. beispielsweise elektromagnetische Strahlung aus dem infraroten, sichtbaren und/oder ultravioletten Spektralbereich reflektieren. Dazu kann der Marker mit einer Oberfläche versehen sein, die entsprechende Reflektionseigenschaften besitzt. Es ist auch möglich, dass ein Marker elektromagnetische Strahlung bzw. Wellen reflektiert und/oder emittiert, die im Bereich der Radiofrequenzen oder bei Ultraschallwellenlängen liegt bzw. liegen. Ein Marker besitzt vorzugsweise sphärische Form bzw. eine kugelähnliche Gestalt und kann daher als Markerkugel bezeichnet werden, Marker können aber auch eine eckige, beispielsweise würfelige Gestalt aufweisen.

Ein Referenzstern bezeichnet eine Einrichtung, an der mehrere, vorteilhaft drei Marker angebracht sind. Die Marker sind dabei ortsfest und vorteilhaft lösbar an dem Referenzstern angebracht, so dass eine bekannte (vorteilhaft feste) relative Lage der Marker zueinander entsteht. Die relative Lage der Marker zueinander kann für jeden im Rahmen eines chirurgischen Navigationsverfahrens verwendeten Referenzstern individuell unterschiedlich sein, um anhand der relativen Lage der Marker zueinander eine Identifikation des dazugehörigen Referenzstems durch ein chirurgisches Navigationssystem zu ermöglichen. Damit können dann auch die Gegenstände (z. B. Instrumente und/oder Körperteile) identifiziert bzw. voneinander unterschieden werden, an welche der Referenzstern angebracht ist. Der Referenzstern dient dazu, in einem chirurgischen Navigationsverfahren eine Mehrzahl von Markern an einem Gegenstand (beispielsweise einem Knochen oder einem medizinischen Instrument) anzubringen, um die Lage des Gegenstandes im Raum (d.h. seine Position und/oder Ausrichtung) erfassen zu können. Ein solcher Referenzstern umfasst insbesondere eine Anbringmöglichkeit an dem Gegenstand (beispielsweise eine Schelle und/oder ein Gewinde) und/oder ein Halteelement, das für einen Abstand der Marker von dem Gegenstand sorgt (um insbesondere die Sichtbarkeit der Marker für eine Markererfassungseinrichtung zu unterstützen) und/oder mit dem Halteelement mechanisch verbundene Markerhalter, an die die Marker angebracht werden können. Wo es aus dem Zusammenhang klar wird, kann der Begriff Referenzstern auch einen Referenzstern mit wenigstens einem daran angebrachten Marker bezeichnen. Ein solches System aus einem Referenzstern und wenigstens einem Marker kann auch Markerstern genannt werden.

Eine Markervorrichtung umfasst mindestens einen Marker, vorzugsweise mindestens 2 oder 3 Marker und kann z.B. ein Referenzstern, ein Pointer und/oder ein einzelner oder mehrere Marker sein. Die am Werkzeug (ortsfest) angebrachte Markervorrichtung wird hierin auch als Werkzeug-Markervorrichtung bezeichnet. Die an der Körperstruktur (ortsfest) angebrachte Markervorrichtung wird hierin auch als Körperstruktur-Markervorrichtung bezeichnet.

Die Erfassung von Landmarken mittels Pointer ist zeitaufwändig und verlängert die Operationszeit und somit die Belastung für den Patienten.

Im Falle der Resektion des distalen Endes des Femurs, um dieses durch ein Implantat zu ersetzen, werden bestimmte Größeninformationen über das distale Ende des Femurs benötigt. Diese werden herkömmlicherweise so erfasst, dass die Condylen mit einem Pointer abgefahren werden, um jeweils den distalsten Punkt zu erfassen. Entsprechend wird vorgegangen, um auf der posterior gelegenen Seite der Condylen den jeweils am weitesten posterior liegenden Punkt zu erfassen. Diese Vorgehensweise ist zeitaufwändig.

WO 2007/085085 A1 beansprucht die Bestimmung eines Referenzpunktes in einer digitalisierten Ebene. Der Referenzpunkt ist der entfernteste Punkt des Femurs innerhalb der digitali sierten Ebene. Diese Druckschrift verlässt sich dabei auf die Bestimmung des Punktes mittels einer Datenbank. Die vorliegende Erfindung bestimmt diesen Punkt nicht, wie weiter unten näher ausgeführt wird.

EP 1 889 584 A1 betrifft die Bestimmung eines Drehzentrum eines Implantats mittels eines speziellen Bestimmungsgeräts, das eine sphärische Oberfläche aufweist.

Aufgabe der Erfindung ist es, das Erfassen die Knochenanatomie beschreibender Größen zu erleichtern und insbesondere zu beschleunigen. Insbesondere ist es Aufgabe der Erfindung, eine korrekte Ausrichtung eines Werkzeugs relativ zu einer Körperstruktur zu unterstützen. Vorstehende Aufgabe wird durch die Gegenstände der unabhängigen Ansprüche gelöst. Vorteilhafte Weiterbildungen gehen aus den Unteransprüchen hervor.

Vorteilhaft wird ein Werkzeug zum Erfassen von Ebenen bereitgestellt, die insbesondere an anatomischen Körperstrukturen, insbesondere Knochen (z.B. Femur) anliegen. Die Ebenen sind insbesondere durch anatomische Landmarken definierbar.

Bei einer Landmarke handelt es sich um einen definierten, charakteristischen Punkt einer anatomischen Struktur, der bei der gleichen anatomischen Struktur mehrerer Patienten stets identisch oder mit hoher Ähnlichkeit wiederkehrt. Typische Landmarken sind beispielsweise die Epikondylen eines Oberschenkelknochens oder die Spitzen der Querfortsätze bzw. des Dornfortsatzes eines Wirbels.

Die Ebenen (genauer deren Lage oder relative Lage zueinander) beschreiben -insbesondere eine bestimmte Formeigenschaft der Körperstruktur (z.B. des Femurs) oder repräsentieren diese Form, die einem Chirurgen wichtige Informationen über die bevorzugte Gestalt und Größe eines Implantats geben. Sie werden deshalb hierin auch als Formrepräsentanten bezeichnet.

Formrepräsentanten repräsentieren einen charakteristischen Aspekt der Form der anatomischen Struktur. Beispiele für Formrepräsentanten sind Geraden, Ebenen oder geometrische Figuren. Geometrische Figuren können eindimensional sein, wie z. B. Achsen oder Kreisbögen, zweidimensional sein, wie z. B. Vielecke und Kreise, oder dreidimensional sein, wie z. B. Quader, Zylinder und Kugeln. Die relative Lage zwischen den Formrepräsentanten kann in Bezugssystemen, z. B. durch Koordinaten oder Vektoren, beschrieben werden oder kann durch geometrische Größen, wie z. B. Länge, Winkel, Fläche, Volumen und Verhältnisse beschrieben werden. Die charakteristischen Aspekte, die durch die Formrepräsentanten repräsentiert werden, sind z. B. Symmetrieeigenschaften, die z. B. durch eine Symmetrieebene repräsentiert werden. Weiter ist ein charakteristischer Aspekt z. B. die Erstreckungsrichtung der anatomischen Struktur, die beispielsweise durch eine Längsachse repräsentiert wird. Weiter ist ein charakteristischer Aspekt z. B. die Querschnittsform einer anatomischen Struktur, die beispielsweise durch eine Ellipse dargestellt wird. Weiter ist ein charakteristischer Aspekt beispielsweise die Oberflächengestalt eines Teils der Struktur, die beispielsweise durch eine Ebene oder eine Halbkugel repräsentiert wird. Der charakteristische Aspekt stellt insbesondere eine Abstraktion der tatsächlichen Form oder eine Abstraktion einer Eigenschaft der tatsächlichen Form (wie z. B. die Symmetrieeigenschaft oder Längestreckung) dar. Der Formrepräsentant repräsentiert insbesondere diese Abstraktion.

Um die Ebenen zu erfassen, werden erfindungsgemäß Anlageflächen bereitgestellt, die ebenfalls plan (eben) sind. Die Anlageflächen können aber auch gekrümmt sein, und z.B. konkave Abschnitte aufweisen, um prominente Knochenteile aufzunehmen. Vorzugsweise umfasst das Werkzeug zwei Anlageflächen, nämlich eine erste und zweite Anlagefläche. Es kann aber auch mehr Anlageflächen umfassen. Die Anlageflächen haben eine vorbestimmte (bekannte) und ortsfeste relative Lage zueinander, insbesondere schließen die durch die Anlageflächen definierten Ebenen bevorzugt einen vorbestimmten (bekannten) Winkel relativ zueinander ein. Der Winkel kann stumpf oder spitz sein. Vorzugsweise ist es ein rechter Winkel. Diejenige Ebene, in der die insbesondere plane Anlagefläche liegt, ist durch diese Anlagefläche definiert.

Das Werkzeug umfasst weiter eine Markervorrichtung. Diese Markervorrichtung hat eine ortsfeste (und bekannte) Lage relativ zu der ersten und zweiten Anlagefläche. Die Markervorrichtung kann lösbar sein. Sie ist aber im verbundenen Zustand, wie gesagt, bevorzugt ortsfest und relativ zu der ersten und zweiten Anlagefläche fixiert.

Das Werkzeug hat bevorzugt eine L-förmige Gestalt. Dies bedeutet insbesondere, dass sich die zwei Anlageflächen nicht (überschneidend) kreuzen. Dies hat den Vorteil, dass das Werkzeug in den häufig sehr begrenzten und schwer zugänglichen Raum zwischen den Körperstrukturen eingeführt werden kann. Ein beengter Raum ist beispielsweise zwischen Femur und Tibia gegeben.

Vorteilhaft umfasst zumindest eine der Anlageflächen, beispielsweise die zweite Anlagefläche, eine Aussparung. Diese Aussparung unterbricht vorzugsweise ein stirnseitiges Ende der Anlagefläche, so dass sich zwei stirnseitige Endteile ergeben, zwischen denen sich die Aussparung befindet. Die Aussparung erstreckt sich vorzugsweise von dem stirnseitigen Ende in Richtung auf die andere Anlagefläche. Beispielsweise erstreckt sich die Aussparung von dem stirnseitigen Ende der zweiten Anlagefläche, das von der ersten Anlagefläche abgewandt ist, in Richtung auf die erste Anlagefläche. Diese Erstreckung der Aussparung wird als eine Erstreckung in Längsrichtung bezeichnet. Diese Erstreckung der Aussparung in Längsrichtung betrifft vorzugsweise den Großteil der Länge der Anlagefläche (z.B. zweiten Anlagefläche), beispielsweise mehr als 50%, 70%, 80% oder 90% der Länge der Anlagefläche oder durchläuft diese komplett und endet in der ersten Anlagefläche. Die Breite der Aussparung nimmt vorzugsweise mehr als ¼ und/oder weniger als ¾ der Gesamtbreite der Anlagefläche ein, in der sich die Aussparung befindet. Vorzugsweise ist die Aussparung größer als 1 cm und/oder kleiner als 10 cm, insbesondere größer als 2 cm und/oder kleiner als 8 cm.

Die Aussparung dient beispielsweise dazu, die zwei durch die Aussparung getrennten Teile der Anlagefläche jeweils an den medialen und den lateralen, vorzugsweise posterioren, Teil eines Condyluse des Femurs anzulegen. Die Aussparung lässt dabei Raum für die Kreuzbänder. Die Breite der Aussparung ist also vorzugsweise so ausgebildet, um die Kreuzbänder aufnehmen zu können.

Vorzugsweise ist an einer der Anlageflächen ein Haltegriff angebracht. Vorzugweise ist der Haltegriff an derjenigen Seite der Anlagefläche angebracht, die von der anderen Anlagefläche weg weist. Vorzugsweise ist eine Markervorrichtung an derselben Anlagefläche angebracht, wo auch der Haltegriff angebracht ist.

Vorzugsweise erstreckt sich die Markervorrichtung, die an einer der Anlageflächen angebracht ist, so von dieser Anlagefläche weg, dass sie sich auch von der anderen Anlagefläche weg erstreckt. Die Markervorrichtung erstreckt sich also insbesondere von beiden Anlageflächen weg.

Vorzugsweise umfasst das Werkzeug an den aufeinander zugewandten Seiten der Anlageflächen Drucksensoren. Die Drucksensoren sind vorzugsweise in vorbestimmten Bereichen vorgesehen, in denen bei korrektem Anlegen des Werkzeugs an den Knochen ein Druckkontakt mit dem Knochen erwartet wird. Vorzugsweise sind diese Drucksensoren auf diese Bereiche beschränkt. Vorzugsweise umfasst das Werkzeug eine Vorrichtung zur Übermittlung des Drucksensorsignals zu einem Detektor, der insbesondere Bestandteil eines Navigationssystems ist. Gemäß einer weiteren Ausführungsform sind mehrere Drucksensoren in einer Anlagefläche vorgesehen, um so flächig aufgelöst den Druck zu erfassen. Auf diese Art und Weise kann bestimmt werden, ob der Bereich, in dem ein Druck erfasst wurde, mit dem zu erwartenden Bereich übereinstimmt oder nicht. Diese Drucksignale können dann übermittelt werden, um so mit Hilfe des Navigationssystems insbesondere Hinweissignale für den Operateur zu erzeugen, ob in den erwarteten Bereichen der Anlagefläche ein Druckkontakt gegeben ist, oder der Druckkontakt in anderen Bereichen gegeben ist. Dadurch wird die Handhabung des Werkzeugs für den Operateur erleichtert. Daten, die die zu erwartenden Bereiche beschreiben, in denen der Druck auftreten soll, können dem Navigationssystem bereitgestellt werden. Die Daten können insbesondere aus Röntgenaufnahmen oder CT-Bildern gewonnen werden und dem Navigationssystem zugeführt werden. Der erwartete Bereich ist insbesondere ein Bereich, für den ein Kontakt mit einem prominenten Teil des Knochens, insbesondere einer prominenten Landmarke, z.B. aufgrund von Knochendaten erwartet wird. Knochendaten beschreiben die Form des Knochens, z.B. basierend auf Röntgen- und CT-Aufnahmen.

Die Erfindung betrifft weiter ein Datenverarbeitungsverfahren, das insbesondere mit Hilfe eines Computers durchgeführt wird. Die zu verarbeitenden Daten werden also vorzugweise einem Computer zugeführt, der diese dann verarbeitet (z.B. einer Berechnung oder Bestimmung durchführt).

Das erfindungsgemäße Verfahrens ist also insbesondere ein Datenverarbeitungsverfahren. Das Datenverarbeitungsverfahren wird bevorzugt durch technische Mittel, insbesondere einem Computer, durchgeführt. Der Computer umfasst insbesondere einen Prozessor und insbesondere einen Speicher, um insbesondere elektronisch die Daten zu verarbeiten. Insbesondere werden die beschriebenen Schritte des Berechnens von einem Computer ausgeführt. Schritte des Definierens von z. B. Bereichen oder Werten sind insbesondere Schritte des Festlegens von Daten im Rahmen des technischen Datenverarbeitungsverfahrens, insbesondere im Rahmen eines Programms. Schritte des Änderns stellen insbesondere eine Änderung der Daten mittels des Computers dar. Schritte des Ermittelns umfassen insbesondere die Abfrage von Werten, die an einer Schnittstelle des Computers anliegen und die durch technische Mittel, wie z. B. einer Abtasteinrichtung, erzeugt wurden. Diese Werte werden insbesondere von der Schnittstelle in Daten umgewandelt, die von dem Computer verarbeitet werden können.

Werden hierin Daten, Regionen, Bereiche oder Bilder "bereitgestellt", so bedeutet dies, dass sie für die Nutzung durch das erfindungsgemäße Verfahren bereit sind. Diesen Zustand des "Bereitstehens" können die Daten z.B. durch Erfassen der Daten, (z.B. durch Analysegeräte) oder durch Eingeben der Daten, (z.B. über Schnittstellen) erreichen. Die Daten können diesen Zustand auch dadurch haben, dass sie in einem Speicher (z.B. ROM, CD, Festplatte) abgespeichert und so für eine Verwendung im Rahmen des erfindungsgemäßen Verfahrens bereit stehen. Vor dem Bereitstellen, insbesondere vor dem Abspeichern, können die Daten, auch in einem Schritt des Verfahrens bestimmt, insbesondere berechnet worden sein.

Die vorliegende Erfindung betrifft ein Datenverarbeitungsverfahren zum Bestimmen der Lage des Werkzeugs relativ zur Körperstruktur. Beim Anlegen des Werkzeugs an die Körperstruktur, insbesondere Knochen, ist die Lage veränderlich. Insbesondere ist die Lage solange veränderlich, bis ein Lagekriterium erfüllt ist, wie weiter unten näher erläutert wird.

Weiter werden Werkzeug-Markerlagedaten bereitgestellt. Diese Markerlagedaten beschreiben die Lage der Werkzeug-Markervorrichtung. Diese Lage wird beispielsweise in einem Bezugssystem eines Navigationssystems, das die Marker der Werkzeug-Markervorrichtung detektiert, beschrieben.

Die Lage, die durch die Werkzeug-Markerlagedaten beschrieben wird, kann sich im Lauf der Zeit ändern. Insbesondere können die Werkzeug-Markerlagedaten zu einem bestimmten Zeitpunkt so sein, dass das weiter unten beschriebene Lagekriterium erfüllt ist. Mithilfe Werkzeug-Markerlagedaten ist somit insbesondere auch eine Änderung der Lage der Werkzeug-Markervorrichtung erfassbar. Insbesondere beschreiben die Werkzeug-Markerlagedaten die Lagen der Werkzeug-Markervorrichtung zu verschiedenen Zeitpunkten.

Weiter werden vorzugsweise Körperstruktur-Markerlagedaten bereitgestellt. Diese beschreiben die Lage der Körperstruktur-Markervorrichtung insbesondere in einem Bezugssystem des Navigationssystems, insbesondere in demselben Bezugssystem des Navigationssystems, in dem auch die Lage der Werkzeug-Markervorrichtung durch die Werkzeug-Markerlagedaten beschrieben wird. Auch die Lage der Körperstruktur-Markervorrichtung kann sich ändern und die Körperstruktur-Markerlagedaten beschreiben insbesondere die sich möglicherweise ändernde Lage zu verschiedenen Zeitpunkten. Vorzugsweise beschreiben sie die Lagen zu denselben Zeitpunkten, wie die Werkzeug-Markerlagedaten die Lagen der Werkzeug-Markervorrichtung beschreiben.

Weiter werden Formdaten (insbesondere Achsendaten) bereitgestellt, die die Lage eines Formrepräsentanten, insbesondere einer Achse, insbesondere der Längsachse des Knochens (z.B. mechanische Achse des Femurs) insbesondere relativ zur Körperstruktur-Markervorrichtung und/oder im Bezugssystem des Navigationssystems beschreiben. Auch diese Formdaten, insbesondere Achsendaten können mittels eines Pointers erfasst und dann dem erfindungsgemäßen Verfahren bereitgestellt werden.

Ist das Lagekriterium erfüllt, so werden diejenigen Werkzeug-Markerlagedaten und diejenigen Körperstruktur-Markerlagedaten bestimmt, die gegeben sind, wenn das Kriterium erfüllt ist. Insbesondere werden diese abgespeichert, die zu dem Zeitpunkt gegeben sind, als das Lagekriterium erfüllt ist oder war. Diejenigen Werkzeug-Markerlagedaten, die dieses Lagekriterium in der oben genannten Weise verfüllen, werden hierin als Kriteriums-Werkzeug-Markerlagedaten bezeichnet. Diejenigen Körperstruktur-Markerlagedaten, die das Lagekriterium in der oben genannten Weise erfüllen, werden Kriteriums-Körperstruktur-Markerlagedaten genannt.

Weiter werden vorzugsweise Körperstrukturdaten bereitgestellt, die die relative Lage der Körperstruktur-Markervorrichtung relativ zu zumindest einem Teil der Körperstruktur, insbesondere zu einem Teil der Oberfläche der Körperstruktur beschreiben.

Vorzugsweise wird basierend auf den Kriteriums-Werkzeug-Markerlagedaten und den Kriteriums-Körperstruktur-Markerlagedaten sowie den Werkzeugdaten und den Körperstrukturdaten die relative Lage der ersten und/oder zweiten Anlagefläche relativ zu zumindest dem Teil der Oberfläche der Körperstruktur berechnet, dessen relative Lage relativ zur Körperstruktur-Markervorrichtung durch die Körperstrukturdaten bestimmt ist. Auf diese Art und Weise lässt sich bestimmen, wie die erste und/oder zweite Anlagefläche relativ zur Körperstruktur liegt, wenn das Lagekriterium erfüllt ist. Alternativ hierzu oder zusätzlich hierzu kann basierend auf den Formdaten, den Werkzeugdaten sowie den Kriteriums-Werkzeug-Markerlagedaten und den Kriteriums-Körperstruktur-Markerlagedaten die relative Lage der ersten und/oder zweiten Anlagefläche relativ zu dem Formrepräsentanten (z.B. der mechanischen Achse) bestimmt werden, die gegeben ist, wenn das Lagekriterium erfüllt ist.

Wie oben ausgeführt, kann somit bestimmt werden, wie, falls das Lagekriterium erfüllt ist, die erste und/oder zweite Anlagefläche relativ zu dem Formrepräsentanten (mechanischer Achse) und/oder relativ zu der Körperstruktur (insbesondere relativ zu einem Teil der Oberfläche der Körperstruktur) liegt, wenn das Lagekriterium erfüllt ist. Diese Informationen erfüllen insbesondere für den Operateur denselben Zweck, wie die Bestimmung der Lage der Gelenklinien relativ zur Körperstruktur oder relativ zur mechanischen Achse. Das beschriebene Datenverarbeitungsverfahren erlaubt jedoch eine einfachere Bestimmung dieser wesentlichen Lageinformationen für den Operateur.

Die vorliegende Erfindung betrifft insbesondere ein Datenverarbeitungsverfahren zum Bestimmen eines Abstandes. Dieser bestimmte Abstand kann insbesondere (auch) zur Auswahl eines Implantats verwendet werden.

Der Abstand wird hierin als Quer-Abstand bezeichnet. Der zu bestimmende Abstand liegt zwischen einer ersten Landmarke (z.B. Anterior-Cortex-Landmarke des Femurs) und einer der Anlageflächen des Werkzeugs (insbesondere der zweiten Anlagefläche). Diese Anlagefläche liegt vorzugsweise auf derjenigen Seite des Knochens, die der genannten ersten Landmarke gegenüberliegt. Anders ausgedrückt, liegt somit zwischen der ersten Landmarke und der Anlagefläche der zweiten Anlagefläche) der Knochen. Dadurch, dass die (erste und/oder zweite) Anlagefläche bevorzugt plan ist, ist ein insbesondere punktförmiger Kontaktbereich zwischen der Anlagefläche und einem vorstehenden Bereich des Knochens, der insbesondere einer Landmarke entspricht, gegeben.

Dem Datenverarbeitungsverfahren werden vorzugsweise eine Reihe von Daten bereitgestellt, so dass es hieraus den Querabstand bestimmten kann. Vorzugsweise werden die genannten Werkzeugdaten bereitgestellt, die die relative Lage der ersten und zweiten Anlagefläche des Werkzeugs zueinander und/oder relativ zur Werkzeug-Markervorrichtung beschreiben. Sollte das Werkzeug weitere Anlageflächen umfassen, so wird deren Lage vorzugsweise ebenfalls durch die Werkzeugdaten beschrieben.

Weiter werden vorzugsweise Landmarkendaten bereitgestellt. Diese beschreiben insbesondere die Lage der ersten Landmarke (z.B. die Anterior-Cortex-Landmarke). Diese Lage kann beispielsweise ebenfalls in dem zuvor erwähnten Bezugssystem des Navigationssystems beschrieben werden. Die Landmarkendaten können beispielsweise mittels eines Pointers erfasst und dann dem erfindungsgemäßen Verfahren bereitgestellt werden.

Basierend auf den bereitgestellten Daten wird mittels des Computers bestimmt, ob das Werkzeug ein Lagekriterium erfüllt, das die Lage des Werkzeugs relativ zu dem Formrepräsentanten, insbesondere zu der Achse (z.B. der mechanischen Achse des Femurs) betrifft. Beispielsweise ist das Lagekriterium so, dass eine der Anlageflächen, beispielsweise die zweite Anlagefläche eine bestimmte Orientierung relativ zum Formrepräsentanten, insbesondere zur Achse einnimmt, insbesondere parallel zu der Achse ist, und/oder dass die Achse normal zu einer durch die Anlageflächen definierten Ebene ist.

Wird eine die relative Lage zwischen der ersten Landmarke und zumindest einer der Anlageflächen, insbesondere der zweiten Anlagefläche beschreibende Größe (im Folgenden Relativgröße genannt), insbesondere Relativpositionen, Relativwinkel oder abstand, ein Abstand, insbesondere der Querabstand zwischen der ersten Landmarke und der zweiten Anlagefläche bestimmt, so ist das Lagekriterium vorzugsweise so, dass die Achse normal zu der durch die erste Anlagefläche definierten Ebene sein soll.

Um zu überprüfen, ob das Lagekriterium erfüllt ist, wird beispielsweise basierend auf den Werkzeugdaten und den Werkzeug-Markerlagedaten die Lage der ersten Anlagefläche berechnet. Ist diese Lage dann bekannt, kennt man auch die Lage der (ersten) Ebene, die durch die erste Anlagefläche definiert ist. Aus den Achsendaten ist die Lage der Achse (z.B. der mechanischen Achse des Femurs) bekannt, so dass man die relative Lage der Achse relativ zu der (ersten) Ebene bestimmen kann. Insbesondere kann man bestimmen, ob die Achse normal zu der (ersten) Ebene ist oder nicht. Vorzugsweise wird die relative Lage der ersten und/oder zweiten Anlagefläche relativ zu der Markervorrichtung durch die Lage der Normalachse der ersten und/oder zweiten Anlagefläche relativ zu der Markervorrichtung mittels den Werkzeugdaten beschrieben.

Ist das Lagekriterium erfüllt, so wird dann die Relativgröße, insbesondere der Quer-Abstand berechnet. Insbesondere werden die Markerlagedaten, die zu dem Zeitpunkt gegeben sind oder waren, als das Lagekriterium erfüllt ist oder war, zur Berechnung der Relativgröße, insbesondere des Quer-Abstandes verwendet.

Gemäß einer Ausführungsform werden dabei dem Datenverarbeitungsverfahren sich möglicherweise ständig ändernde Markerlagedaten bereitgestellt, und diejenigen Markerlagedaten werden herausgegriffen, für die basierend auf den Achsendaten die Erfüllung des Lagekriteriums angezeigt wird.

Die bereitgestellten Achsendaten können wiederum aus anderen Daten berechnet werden. Insbesondere kann die Achse z.B. im Falle des Femurs aus einem Rotationspunkt, um den sich der Schenkelhals dreht, und dem distalen Achsenpunkt, der insbesondere mit dem Ende des Markerkanals und/oder einer Landmarke, wie Trochlea Ossis Femoris übereinstimmt, bestimmt werden. Die Achse ergibt sich aus der Verbindung des Rotationspunktes mit der Landmarke. Auch diese Bestimmung kann Bestandteil des Datenverarbeitungsverfahrens sein. Dem Datenverarbeitungsverfahren werden dann die hierzu notwendigen Punkte oder Landmarken (z.B. Rotationspunkt und Trochlea Ossis Femoris) bereitgestellt.

Vorzugsweise wird die Lage des Schnittpunkts der Achse mit einer der Anlageflächen, insbesondere der ersten Anlagefläche berechnet. Diese Lage kann in dem vorgenannten Bezugssystem des Navigationssystems berechnet werden. Der so berechnete Schnittpunkt kann wiederum verwendet werden, um basierend auf ihm eine Gelenklinie zu berechnen. Die Gelenklinie dient dem Chirurgen ebenfalls als Anhaltspunkt für die Platzierung eines Implantats. Die Gelenklinie wird so bestimmt, dass von dem genannten Schnittpunkt ein Lot auf die zweite Anlagefläche gefällt wird. Das Lot entspricht der Gelenklinie

Der oben erwähnte Quer-Abstand wird vorzugsweise so mittels des Datenverarbeitungsverfahrens berechnet, dass ein Lot von der ersten Landmarke auf die zweite Anlagefläche gefällt wird. Die Länge des Lots ist dann der Querabstand.

Neben dem Querabstand oder alternativ zu dem Querabstand wird vorzugsweise auch eine andere Relativgröße, insbesondere ein Längs-Abstand berechnet. Dies ist der kürzeste Abstand von dem Quer-Lot und dem genannten Schnittpunkt, der sich aus der Achse und der ersten Anlagefläche ergibt. Dieser Längs-Abstand dient ebenfalls dem Chirurgen zur Auswahl des Implantats.

Neben dem oben genannten Lagekriterium, wird vorzugsweise überprüft, ob eine weitere Bedingung erfüllt ist. Diese Bedingung ist der Kontakt zwischen dem Werkzeug und dem Knochen, insbesondere eines vorbestimmten Teils (zumindest einer der beiden Anlageflächen) des Werkzeugs mit einem gewünschten (vorbestimmten) Teil des Knochens (einer prominenten Landmarke). Zu diesem Zweck werden dem Datenverarbeitungsverfahren vorzugsweise Kontaktdaten bereitgestellt, die anzeigen, ob ein Kontakt gegeben ist oder nicht. Insbesondere zeigen sie an, ob der Kontaktbereich, wie gewünscht, mit einer Landmarke übereinstimmt. Wie oben beschrieben, kann mittels Drucksensoren im Werkzeug der Kontakt bestimmt werden. Bevorzugt zeigen die Drucksensoren den Kontakt in einem vorbestimmten Bereich der Anlageflächen an. Weiter umfassen die Knochendaten bevorzugt Informationen, um überprüfen zu können, ob der Kontakt mit dem gewünschten Teil des Knochens hergestellt wurde. Alternativ kann dies so erfolgen, dass der Bediener (Chirurg) über eine Benutzerschnittstelle (z.B. Tastatur) dem Datenverarbeitungsverfahren mitteilt, dass ein Kontakt zwischen dem Werkzeug und dem Knochen, insbesondere eines vorbestimmten Teils des Werkzeugs mit dem gewünschten Teil des Knochens, hergestellt ist. Sind die Kontaktdaten z.B. in der oben beschriebenen Art und Weise bereitgestellt, so werden sie durch das Datenverarbeitungsverfahren überprüft. Falls die Kontaktdaten einen Kontakt, insbesondere des vorbestimmten Teils des Werkzeugs mit dem gewünschten (vorbestimmten) Teil des Knochens, anzeigen, wird die Berechnung der Relativgröße, insbesondere des Querabstandes durchgeführt. Anders ausgedrückt, diejenigen bereitgestellten Daten werden zur Berechnung des Quer-Abstandes verwendet, die zu dem Zeitpunkt gelten, wenn die Kontaktdaten einen Kontakt anzeigen, insbesondere einen Kontakt des vorbestimmten Teils des Werkzeugs mit dem gewünschten (vorbestimmten) Teil des Knochens anzeigen, und wenn das Lagekriterium erfüllt ist. Ist der vorbestimmte Teil des Werkzeugs, z.B. jeweils ein Teil der ersten und zweiten Anlagefläche mit jeweils einem vorbestimmten Teil des Knochens in Kontakt, so ist dies ein Hinweis darauf, dass das Werkzeug an den Knochen korrekt angelegt wurde.

Die von einem Drucksensor detektierten Signale können beispielsweise über Funk an das Navigationssystem übermittelt werden, um vom erfindungsgemäßen Datenverarbeitungsverfahren verarbeitet zu werden.

Vorzugsweise generiert das Datenverarbeitungsverfahren erste Hinweisinformationen, die bevorzugt (wie alle Hinweisinformationen) mittels eines ersten Hinweissignals ausgegeben werden. Diese Hinweisinformationen beschreiben die Relativgröße, insbesondere den berechneten Quer-Abstand und/oder Längsabstand.

Weiter werden vorzugsweise von dem Datenverarbeitungsverfahren zweite Hinweisinformationen generiert. Diese zweiten Hinweisinformationen beschreiben die Orientierung der zweiten Anlagefläche relativ zur Achse. Auf diese Art und Weise kann einem Bediener (Chirurgen) über Hinweissignale, die die zweite Hinweisinformation anzeigen, ein Hinweis gegeben werden, ob das Lagekriterium erfüllt ist oder nicht.

Weiter ist die Erfindung vorzugsweise auf ein Programm gerichtet, das, wenn es auf einem Computer läuft oder in einen Computer geladen wird, den Computer dazu veranlasst, das Datenverarbeitungsverfahren auszuführen.

Weiter ist die Erfindung vorzugsweise auf ein Navigationssystem gerichtet, das einen Computer umfasst, in den das vorgenannte Programm geladen ist oder auf dem das vorgenannte Programm läuft. Das Navigationssystem umfasst neben dem Computer vorzugsweise eine Detektionsvorrichtung, um die Markervorrichtung des Werkzeuges zu detektieren, um so die Markerlagedaten zu generieren. Weiter umfasst es vorzugsweise eine Benutzerschnittstelle, insbesondere zur Eingabe der Kontaktdaten. Weiter umfasst es vorzugsweise eine Benutzerschnittstelle (Monitor, Lautsprecher etc.), um akustische und/oder visuelle und/oder taktile Hinweissignale auszugeben, wie sie oben erwähnt wurden. Weiter umfasst es vorzugsweise eine Datenschnittstelle, um die weiteren genannten Daten dem Computer zuzuführen. Insbesondere ist ein Speicher vorgesehen, um die genannten Daten abzuspeichern und für das Datenverarbeitungsverfahren bereitzustellen.

Kurzbeschreibung der Figuren:
- Figur 1: zeigt ein Femur aus der A-P-Blickrichtung (Anterior-Posterior- Blickrichtung);
- Figur 2a: zeigt das Femur der Figur 1 mit eingetragener mechanischer Achse;
- Figur 2b: zeigt eine Ansicht des distalen Endes des Femurs aus der distal-proximalen Richtung;
- Figur 3: zeigt wiederum ein Femur aus der A-P-Blickrichtung mit erfindungsgemä- ßem Werkzeug;
- Figur 4: zeigt das Femur aus der M-L-Blickrichtung mit dem erfindungsgemäßen Werkzeug;
- Figur 5: zeigt eine erste Ausführungsform des erfindungsgemäßen Werkzeugs;
- Figur 6: zeigt eine zweite Ausführungsform des erfindungsgemäßen Werkzeugs;
- Figur 7: zeigt ein erfindungsgemäßes System mit Navigationssystem und dem erfin- dungsgemäßen Werkzeug.

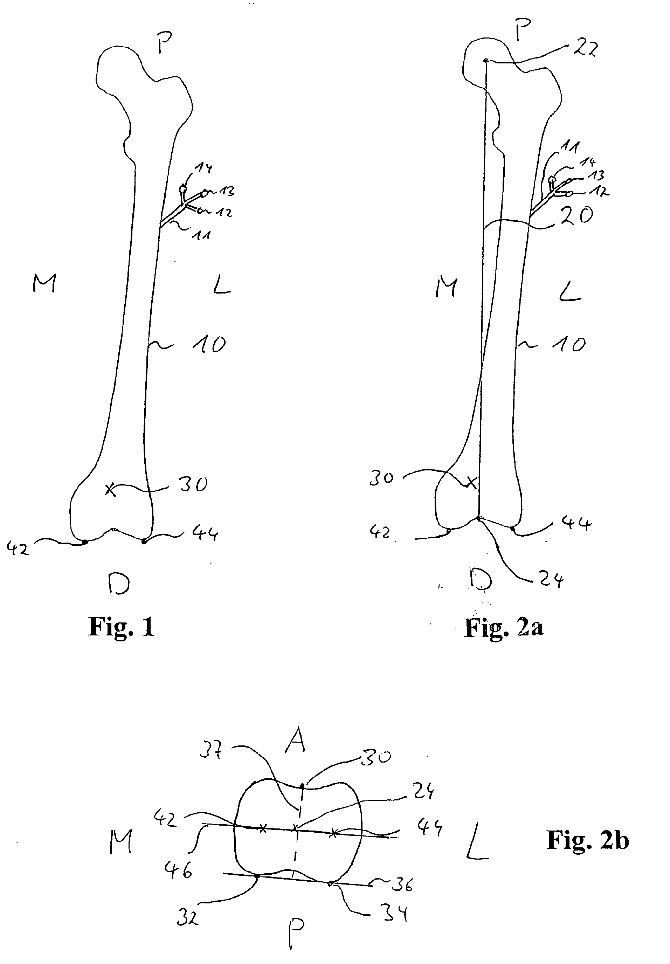

Figur 1 zeigt ein Femur aus der A-P-Blickrichtung. Es handelt sich um ein linkes Femur. Der Buchstabe "M" bedeutet medial. Der Buchstabe "L" bedeutet lateral. Der Buchstabe "P" bedeutet proximal und der Buchstabe "D" bedeutet distal. Soweit hierin der Buchstabe "P" dem Buchstaben "D" gegenüberliegt, bedeutet er proximal. Falls er dem Buchstaben "A" gegenüberliegt, bedeutet er posterior, wobei "A" anterior bedeutet.

Die Lehre der Erfindung betrifft die Erfassung charakteristischer Größen des distalen Endes des Femurs. Hierzu wird im Stand der Technik mittels eines Pointers der Anterior-Cortex-Punkt 30 als Landmarke erfasst. Die Bezugszeichen 42 und 44 bezeichnen jeweils ein medialdistales Ende und lateral-distales Ende der Condylen. Um diese Punkte 42 und 44 zu erfassen, werden im Stand der Technik eine Reihe von Punkten im Bereich der distalen Enden der beiden Condylen mittels eines Pointers abgegriffen, um so jeweils den am weitesten distal liegenden Punkt als Punkt 42 und 44 zu bestimmen. Dies ist allerdings zeitaufwändig. Die Punkte 42 und 44 stellen Beispiele für prominente Landmarken dar, die mit der (ersten) Anlagefläche des Werkzeugs in Kontakt kommen sollen. Durch die Verbindung der Punkte 42 und 44 ergibt sich die Gelenklinie im Falle der Extension. Die Gelenklinie im Fall der Flexion ergibt sich durch die Verbindung der Punkte 32 und 34 (siehe Figur 2b).

Figur 2a zeigt die Bestimmung der mechanischen Achse 20. Hierzu wird das Drehzentrum 22 des Femurs in der Gelenkpfanne in bekannter Weise bestimmt. Dieses Drehzentrum 22 wird mit dem distalen Achsenendpunkt verbunden. Der Drehpunkt 22 wird also mit dem distalen Achsenpunkt 24 verbunden. Der distale Achsenpunkt 24 ist auch in der Figur 2b zu sehen.

Gemäß dem Stand der Technik ist die Gelenklinie für den Operateur wichtig, um die Implantation eines Implantats zu planen, falls das distale Ende des Femurs reseziert wird. Im Vergleich zur Gelenklinie (Extension) 46 proximal liegt eine gestrichelt eingezeichnete Linie 37. Diese ergibt sich in bekannter Weise durch die Verbindung des Arterior-Cortex-Punktes 30 mit der Gelenklinie (Flexion) 36. Die Gelenklinie (Flexion) 36 ergibt sich in ebenfalls bekannter Weise durch die Verbindung zweier Punkte, die auf jeder der beiden Condylen am weitesten posterior liegen. Um diese posterioren Condylen-Punkte 32 und 34 zu ermitteln, wird im Stand der Technik ebenfalls eine Reihe von Punkten auf den Condylen abgetastet und derjenige jeweils ausgewählt, der am weitesten posterior liegt. Auch dies ist zeitaufwändig. Die Punkte 32 und 34 stellen Beispiele für prominente Landmarken dar, die mit der (zweiten) Anlagefläche in Kontakt kommen sollen. Die Gelenklinie 46, die im Falle der Extension gegeben ist, und die Gelenklinie 36, die im Falle der Flexion gegeben ist, stellen für den Operateur wichtige Anhaltspunkte für die Wahl des Implantats dar, insbesondere ihre relative Lage zum Knochen.

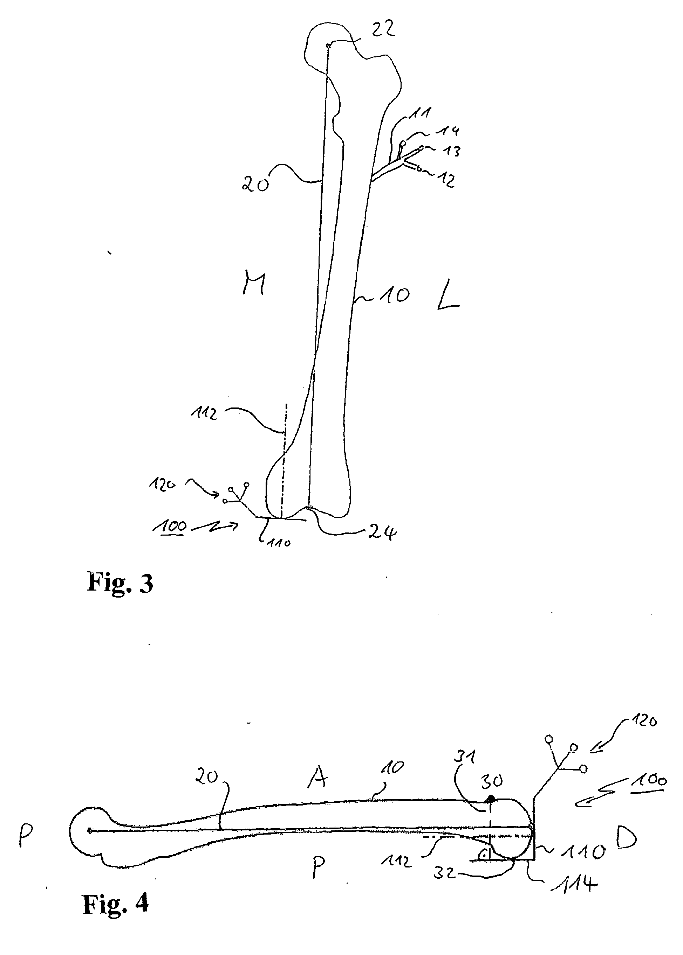

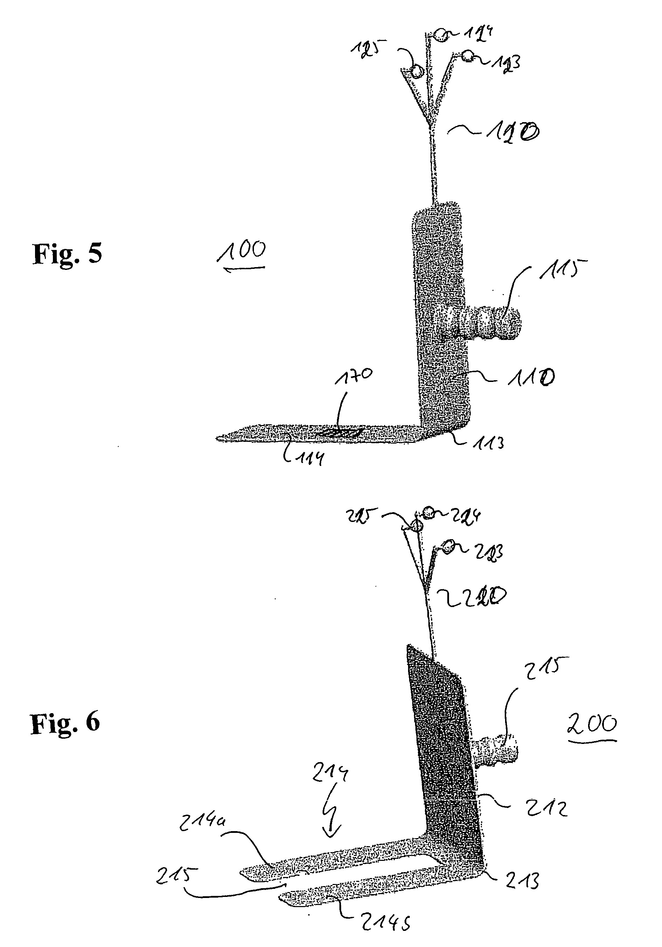

Figur 3 zeigt die Anwendung des erfindungsgemäßen Werkzeuges 100 zur Ermittlung charakteristischer Ebenen des distalen Endes des Femurs. Das Werkzeug 100, das insbesondere eine L-förmige Gestalt hat, wie es in Figur 5 und 6 zu sehen ist, wird mit einer ersten Anlagefläche 110 am medial distalen Ende des Femurs angelegt, so dass die erste Anlagefläche 110 mit dem distalen Condylen-Punkt 42 in Kontakt ist, der in diesem Beispiel die prominente Landmarke darstellt, die in Kontakt mit der ersten Anlagefläche 110 den Kontaktbereich definiert. Das Werkzeug 100 wird dabei weiter so ausgerichtet, dass eine Normale 112 auf die erste Anlagefläche 110, die eben ist, parallel zur mechanischen Achse 20 verläuft.

Figur 4 zeigt die Situation der Figur 3 aus der M-L-Blickrichtung. Das Werkzeug 100 (wie es in Figur 5 gezeigt ist) ist an das Femur 10 in derselben Art und Weise angelegt wie in Figur 3. Weiter ist zu sehen, dass die zweite Anlagefläche 114 am posterioren Ende des medialen Condylus anliegt, und so mit dem Punkt 32, der in Figur 2b zu sehen ist, in Kontakt ist. Der Punkt 32 stellt ein Beispiel für eine prominente Landmarke dar, die in Kontakt mit der zweiten Anlagefläche 114 den Kontaktbereich bestimmt. Im gezeigten Beispiel ist jede der Anlageflächen mit nur einer prominenten Landmarke in Kontakt. Vorzugsweise ist jede Anlagefläche mit mindestens einer (prominenten) Landmarke in Kontakt. Es kann aber auch gewünscht sein, dass mehr als eine Landmarke mit der Anlagefläche in Kontakt ist.

Ebenfalls ist in Figur 3 erkennbar, dass die zweite Anlagefläche 114 parallel zur mechanischen Achse 20 verläuft. Da die beiden Anlageflächen 110 (erste Anlagefläche) und 114 (zweite Anlagefläche) orthogonal zueinander sind, ist somit auch eine Normale 112 der Anlagefläche 110 parallel zur mechanischen Achse.

Ebenfalls in Figur 3 und 4 zu sehen, ist ein Referenzstern 120, der als Werkzeug-Markervorrichtung dazu dient, mittels eines Navigationssystems die Lage des Werkzeugs 100 zu bestimmen.

Weiter ist in den Figuren 1, 2a und 3 ein Referenzstern 11 gezeigt, der als Körperstruktur-Markervorrichtung dient und ortsfest relativ zum Knochen 10 angebracht ist. Der Referenzstern 11 umfasst die Markerkugeln 12, 13 und 14.

Figur 5 zeigt das erfindungsgemäße Werkzeug 100, bei dem die Anlageflächen 110 und 114 eine L-Form bilden und zueinander senkrecht sind. Die beiden Anlageflächen 110 und 114 sind durch einen gekrümmten Bereich 113 miteinander verbunden. Ein Handgriff 115 erlaubt die Handhabung des Werkzeuges 100. Das in Figur 5 gezeigte Werkzeug umfasst einen Referenzstern 120 am stirnseitigen Ende der Anlagefläche 110. Dort steht er in Längserstreckungsrichtung der Anlagefläche 110 ab. Die Breite der Anlagefläche 110 ist insbesondere größer als 1/10 der Länge der Anlagefläche 110 und/oder insbesondere kleiner als die Hälfte der Länge der Anlagefläche 110. Die Breite der Anlagefläche 110 ist insbesondere größer als 1 cm und/oder kleiner als 10 cm. Die Länge der Anlagefläche ist insbesondere länger als 2 cm und/oder kürzer als 15 cm. Die eben genannten Größen gelten insbesondere auch für die zweite Anlagefläche 114. Die Dicke der Anlageflächen ist insbesondere größer als 0,1 mm und/oder kleiner als 5 cm, insbesondere kleiner als 10 mm.

Das Werkzeug umfasst als Materialen insbesondere Metall, Kunststoff und/oder Verbundwerkstoffe und/oder Keramiken.

Die in Figur 5 gezeigte Ausführungsform dient insbesondere zum Anliegen der beiden Anlageflächen an nur eine der beiden Condylen, genauer an ein distales Ende und an ein posteriores Ende einer der Condylen. Vorzugsweise sind die Abmessungen des Werkzeugs 100, das in Figur 5 gezeigt ist, entsprechend gewählt.

Die Lage eines optionalen (und flächig ausgebildeten) Drucksensors ist mit 170 gekennzeichnet.

Figur 6 zeigt eine alternative Ausführungsform des Werkzeuges, das mit dem Bezugszeichen 200 versehen ist. Das Werkzeug 200 hat eine erste Anlagefläche 212 und eine zweigeteilte zweite Anlagefläche 214a und 214b, die im gekrümmten Bereich 213, der die beiden Anlageflächen verbindet oder bereits vor dem gekrümmten Bereich 213 miteinander verbunden sind. Eine Aussparung 215 trennt die beiden Teile der Anlagefläche 214a und 214b. Die Aussparung 215 erstreckt sich von dem stirnseitigen Ende der Anlagefläche 214, das der Anlagefläche 212 abgewandt ist, in Richtung auf die Anlagefläche 212, genauer in Richtung auf den gekrümmten Abschnitt 213 des Werkzeugs 200. Die Aussparung ist insbesondere länglich, das heißt, der Verlauf in Längserstreckungsrichtung in Richtung auf den gekrümmten Verbindungsabschnitt ist länger als der Verlauf der Aussparung in Breitenrichtung. Insbesondere ist der Verlauf der Aussparung in Längsrichtung größer als das Doppelte des Verlaufs in Breitenrichtung und/oder kleiner als das Zehnfache des Verlaufs in Breitenrichtung. Die Aussparung hat eine Breite, die insbesondere größer ist als 1 cm und/oder kleiner ist als 5 cm. Die Aussparung ist insbesondere so gestaltet, dass sie die zwischen den Condylen verlaufenden Kreuzbänder aufnehmen kann, so dass die zweite Anlagefläche zwischen Femur und Tibia eingeführt werden kann, während zwischen den Anlageflächenteilen 214a und 214b die Kreuzbänder aufgenommen werden.

Für die Längen des in Figur 6 gezeigten Werkzeugs 200, insbesondere für die Längen der Anlageflächen 212 und 214 gelten vorzugsweise dieselben Größenangaben, wie diejenigen für die Anlageflächen 110 und 114. Vorzugsweise ist allerdings die Breite der Anlagenflächen 212 und 214 größer als die Breite der Anlageflächen 110 und 114. Insbesondere gelten bezüglich der Breitenrichtung die doppelten oder dreifachen Werte für die Obergrenzen und Untergrenzen für das Werkzeug 200 im Vergleich zum Werkzeug 100.

Der Handgriff 215 erlaubt ebenso wie der Handgriff 115 eine leichtere Handhabung des Werkzeugs. Die Referenzsterne sind in Figur 5 mit dem Bezugszeichen 120 gekennzeichnet und in Figur 6 mit dem Bezugszeichen 220. Die einzelnen Markerkugeln der Markervorrichtung 120 sind mit den Bezugszeichen 123, 124 und 125 in Figur 5 und die der Markervorrichtung 220 mit den Bezugszeichen 223, 224 und 225 in Figur 6 bezeichnet.

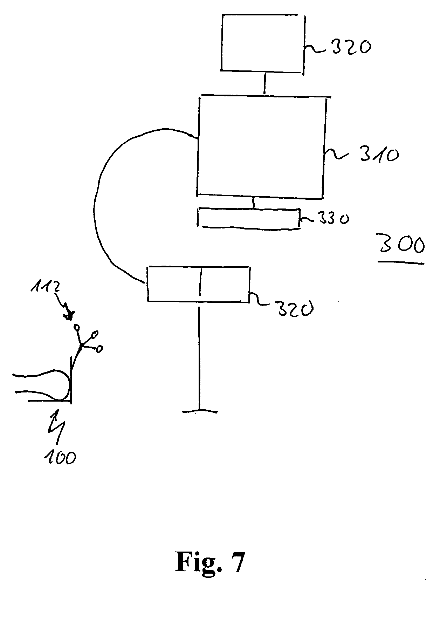

Figur 7 zeigt ein erfindungsgemäßes System, bestehend aus dem Werkzeug 100 (oder 200) und einem Navigationssystem 300. Das Navigationssystem 300 umfasst vorzugsweise einen Computer 310, einen Monitor 320 sowie eine mit dem Computer 310 verbundene Detektionsvorrichtung 325. Die Detektionsvorrichtung detektiert von den Referenzsternen 11 und 120 emittierte oder reflektierte Strahlung. Die durch die Strahlung erzeugten Detektionssignale werden dem Computer 310 zugeführt, der hierauf basierend die Lage des Werkzeugs 100 in einem Bezugssystem berechnet. Die Lage kann insbesondere am Monitor 320 angezeigt werden. Der Monitor 320 kann auch zur Anzeige weiterer Hinweisinformationen genutzt werden. Als Benutzerschnittstelle kann weiterhin eine Tastatur oder eine Maus 330 vorgesehen sein. Auf dem Computer 310 läuft insbesondere das erfindungsgemäße Datenverarbeitungsverfahren. Das Datenverarbeitungsverfahren bestimmt insbesondere die Lage des Werkzeugs und überprüft insbesondere, ob die Normale zur ersten Anlagefläche 110 oder 212) parallel zur mechanischen Achse ist. Die Lage der mechanischen Achse wird entweder über eine Benutzerschnittstelle eingegeben oder es werden Daten eingegeben, die die Bestimmung der Lage der mechanischen Achse erlaubten. Beispielsweise werden dem Navigationssystem die Lage des Drehpunkts 22 und beispielsweise mit Hilfe eines Pointers die Lage des Trochlea-Punktes 24 mitgeteilt. Die bereitgestellten Daten erlauben somit die Bestimmung der Lage der mechanischen Achse. Die somit dem System bereitgestellten Daten erlauben die Bestimmung der Lage des Werkzeugs relativ zur Körperstruktur, insbesondere die Bestimmung der Lage der ersten und/oder zweiten Anlagefläche relativ zur Körperstruktur und/oder relativ zur mechanischen Achse für den Fall, dass das Lagekriterium erfüllt ist. Insbesondere erlauben die zur Verfügung stehenden Daten die Überprüfung, ob dieses Lagekriterium erfüllt ist und insbesondere zu welchem Zeitpunkt dieses Lagekriterium erfüllt ist. Die Daten betreffend die Lage des Werkzeugs (Kriteriums-Werkzeug-Markerlagedaten) und betreffend die Lage der Körperstruktur (des Knochens) (Kriteriums-Körperstruktur-Markerlagedaten), die zu dem Zeitpunkt gegeben sind, wenn das Lagekriterium erfüllt ist oder war, werden dann verwendet, um die relative Lage des Werkzeugs relativ zur Körperstruktur und/oder zum Formrepräsentanten (zur mechanischen Achse) zu bestimmen. Die Informationen über diese relative Lage wird vorzugsweise dem Operateur beispielsweise durch die Benutzerschnittstelle (Monitor 320) als Hinweisinformation mitgeteilt.

Weiter wird dem System vorzugsweise die Lage des Anterior-Cortex-Punktes mitgeteilt. Die so dem Verfahren oder dem System bereitgestellten Daten können genutzt werden, um den Abstand zwischen dem in Figur 4 gezeigten Anterior-Cortex-Punkt 30 und der zweiten Anlagefläche 114 (oder 214) zu bestimmen. Insbesondere wird das Lot (Quer-Lot) von dem Anterior-Cortex-Punkt 30 auf die Anlagefläche 114 gefällt, um den Abstand zwischen dem Anterior-Cortex-Punkt 30 und der Fläche 114 zu bestimmen. Die so ermittelte Größe ist für den Operateur von Bedeutung, um ein geeignetes Implantat auszuwählen, falls das distale Ende des Femurs reseziert wird. Insbesondere dieser Abstand wird am Monitor 320 dargestellt. Das Fällen des Quer-Lots 31 ist in Figur 4 durch eine gestrichelte Linie angedeutet.

Das Navigationssystem bestimmt insbesondere mittels des Referenzsterns auch die Orientierung der ersten Anlagefläche 110 oder 212. Diese Orientierung der Anlagefläche 110 erlaubt dann die Bestimmung der vorgenannten Gelenklinie. Die Gelenklinie wird insbesondere so bestimmt, dass sie in der ersten Anlagefläche 110 liegt und normal zur zweiten Anlagefläche 114 (oder 214) verläuft.

Die vorgenannten Verfahrensschritte werden durch den Computer 310 ausgeführt, der die bereitgestellten Daten verarbeitet, um die gewünschten Größen, insbesondere den Abstand zwischen dem Punkt 30 und der Ebene 114, sowie die Lage der Gelenklinie zu berechnen. Weiter wird vorzugsweise der Abstand zwischen dem Quer-Lot 31 und der ersten Anlagefläche 110 (oder 212) bestimmt und insbesondere angezeigt.

Um die Achsendaten zu gewinnen, kann die mechanische Achse des Femurs beispielsweise durch die folgenden Schritte bestimmt werden:
- Am Femur wird ein Referenzstern angebracht.
- Das Femur wird in der Gelenkpfanne pivotiert, um den Rotationspunkt des Femurkopfes zu errechnen.
- Der distale Achsenendpunkt wird mit einem Pointer aufgenommen.
- Diese beiden Punkte werden verbunden und heißen von nun ab "mechanische Achse".
- Die Anlagefläche 214 wird in den Gelenkspalt eingschoben und damit an der medialen und der lateralen, posterioren Seite des Condylus angelegt.
- Das L-Shape-Device wird soweit eingeschoben, bis die Anlagefläche 212 am distalen Ende des Femurs anliegt.
- Das L-Shape-Device wird nun um die M-L-Achse und um die A-P-Achse rotiert.
- Dabei liegen stets der mediale und der laterale, posteriore Condylus sowie das distale Ende des Femurs (evtl. nur ein Punkt) an den Anlageflächen 214 bzw. 212 an.
- Die Lage der Normalachse zur Anlagefläche 212 wird nun basierend auf den Werkzeugdaten und den Markerlagedaten berechnet und mit der Lage der mechanischen Achse verglichen. Dies geschieht vorzugsweise in zwei Projektionen:

Der Ansicht in M-L-Richtung und der Ansicht in A-P-Richtung
- Sobald die Normalachse in beiden beobachteten Ansichten zugleich zur mechanischen Achse parallel liegt, wird die Position des L-Shape-Devices gespeichert.
- Durch die Position der Anlagefläche 214 ergibt sich eine Ebene durch die beiden posteriorsten Punkte des Femurs. Diese Ebene wird zur internen/externen Rotationsbestimmung des Implantats bzw. der zur Implantation benötigten Knochenschnitte verwendet.
- Durch die Position der Anlagefläche 212 ergibt sich eine Ebene durch den distalsten Punkt des Femurs. Diese Ebene wird zur Navigation des distalen Schnittes verwendet, durch den die Beinlänge, Varus / Valgus sowie Flexion / Extension determiniert werden.

## Patentansprüche

1. Datenverarbeitungsverfahren zum Bestimmen der veränderlichen Lage eines Werkzeugs (100) mit erster und zweiter ebenen Anlagefläche (110, 114; 212, 214) relativ zu einer Körperstruktur (10), wobei die erste Anlagefläche (110, 114) an einer ersten Seite der Körperstruktur (10) anliegt und die zweite Anlagefläche (212, 214) an einer zweiten Seite der Körperstruktur (10) anliegt und die erste und zweite Anlagefläche (110, 114; 212, 214) eine vorbestimmte relative Lage zueinander haben, wobei das Werkzeug eine Werkzeug-Markervorrichtung (120; 220) aufweist, die eine ortsfeste Lage relativ zur ersten und zweiten Anlagefläche (110, 114; 212, 214) hat und wobei das Datenverarbeitungsverfahren von einem Computer durchgeführt wird,
wobei folgende Daten bereitgestellt werden:
- Werkzeug-Markerlagedaten, die die Lage der Werkzeug-Markervorrichtung (120; 220) beschreiben; und Körperstruktur-Makerlagedaten, die die Lage einer an der Körperstruktur (10) angebrachten Körperstruktur-Markervorrichtung (11) beschreiben;
- Formdaten, die die Lage eines Formrepräsentanten der Körperstruktur (10) beschreiben,
**dadurch gekennzeichnet, dass** folgende Datenverarbeitungsschritte durchgeführt werden:
es wird bestimmt, ob die Lage des Formrepräsentanten ein Lagekriterium erfüllt, wobei das Lagekriterium so ist, dass die erste und/oder zweite Anlagefläche (110, 114; 212, 214) eine bestimmte Orientierung relativ zum Formrepräsentanten einnimmt;
die Werkzeug-Markerlagedaten und die Körperstruktur-Markerlagedaten, die gegeben sind, wenn die Erfüllung des Lagekriteriums bestimmt wird, werden bestimmt.

2. Datenverarbeitungsverfahren nach Anspruch 1, bei welchem basierend auf den bestimmten Werkzeug-Markerlagedaten und den bestimmten Körperstruktur-Markerlagedaten sowie den Formdaten und Werkzeugdaten, die die relative Lage der ersten und/oder zweiten Anlagefläche relativ zu der Werkzeug-Markervorrichtung beschreiben, die relative Lage zwischen dem Formrepräsentanten und der ersten und/oder zweiten Anlagefläche bestimmt wird und/oder basierend auf den bestimmten Werkzeug-Markerlagedaten, den bestimmten Körperstruktur-Markerlagedaten, den Werkzeugdaten und Körperstrukturdaten, die die relative Lage der Körperstruktur-Markervorrichtung relativ zu zumindest einem Teil der Körperstruktur beschreiben, die relative Lage zwischen zumindest dem Teil der Körperstruktur und der ersten und/oder zweiten Anlagefläche bestimmt wird.

3. Datenverarbeitungsverfahren nach Anspruch 1 oder 2, bei welchem Werkzeugdaten bereitgestellt werden, die die relative Lage der ersten und zweiten Anlageflächen des Werkzeugs relativ zu der Werkzeug-Markervorrichtung beschreiben, Landmarkendaten bereitgestellt werden, die Lage einer ersten Landmarke in dem Bezugsystem beschreiben, und der Formrepräsentant eine Achse ist und die Körperstruktur ein Knochen ist und basierend auf den bestimmten Werkzeug-Markerlagedaten und bestimmten Körperstruktur-Markerlagedaten sowie den Werkzeugdaten wird eine die relative Lage zwischen der ersten Landmarke und zumindest eine der Anlagefläche beschreibende Größe berechnet werden, wobei die Größe ein quer zu der Achse verlaufender Quer-Abstand zwischen der ersten Landmarke und der zweiten Anlagefläche ist.

4. Datenverarbeitungsverfahren nach Anspruch 3, bei welchem die Lage des Schnittpunktes der Achse mit der ersten Anlagefläche berechnet wird, wobei eine als Gelenklinie bezeichnete Linie berechnet wird, indem vom berechneten Schnittpunkt ein erstes Lot auf die zweite Anlagefläche gefällt wird.

5. Datenverarbeitungsverfahren nach Anspruch 2 oder 3, bei welchem zur Bestimmung des Quer-Abstandes ein zweites Lot von der ersten Landmarke auf die zweite Anlagefläche gefällt wird; und/oder bei welchem ein als Längs-Abstand bezeichneter Abstand, der längs zur Achse verläuft, zwischen dem zweiten Lot und dem ersten Lot oder dem Schnittpunkt berechnet wird.

6. Datenverarbeitungsverfahren nach einem der Ansprüche 2 bis 5, bei welchem dem Datenverarbeitungsverfahren weiter Kontaktdaten bereitgestellt werden, die anzeigen, ob die Anlageflächen mit dem Knochen in Kontakt sind und die Berechnung des Quer-Abstandes und/oder des Längsabstandes durchgeführt wird, falls der Kontakt durch die Kontaktdaten angezeigt wird.

7. Datenverarbeitungsverfahren nach einem der Ansprüche 2 bis 6, bei welchem erste Hinweisinformationen generiert werden, die den berechneten Quer-Abstand und/oder Längsabstand anzeigen und/oder bei welchem zweite Hinweisinformationen ausgegeben werden, die einen Hinweis zu der Orientierung der zweiten Anlagefläche relativ zur Achse geben.

8. Programm, das, wenn es auf einem Computer läuft oder in einen Computer geladen wird, den Computer veranlasst, das Datenverarbeitungsverfahren nach einem der Ansprüche 2 bis 7 auszuführen.

9. Navigationssystem mit einem Computer, in dem das Programm nach Anspruch 8 geladen ist oder auf dem Computer läuft.

10. System mit dem Navigationssystem nach Anspruch 9 und mit einem Werkzeug (100) zum Erfassen von durch anatomische Landmarken definierten Ebenen
mit einer ersten und zweiten ebenen Anlagefläche (110, 114; 212, 214) zum Anlegen an eine Körperstruktur (10),
wobei die erste und zweite Anlagefläche (110, 114; 212, 214) eine vorbestimmte relative Lage zu einander haben, und
mit einer Werkzeug-Markervorrichtung (120; 220), die eine ortsfeste Lage relativ zu der ersten und zweiten Anlagefläche (110, 114; 212, 214) hat.

11. System nach Anspruch 10, wobei das Werkzeug eine L-förmige Gestalt hat, wobei die erste und zweite Anlagefläche (110, 114; 212, 214) das L bilden.

12. System nach Anspruch 10 oder 11, wobei die zweite Anlagefläche (214) ein Aussparung aufweist, die zwischen zwei stirnseitigen Enden (214a, 214b) der zweiten Anlagefläche, die von der ersten Anlagefläche (212) abgewandt sind, beginnt und sich in Richtung auf die erste Anlagefläche (212) über einen Großteil der Länge der zweiten Anlagefläche (214) in Längsrichtung erstreckt.

13. System nach einem der Ansprüche 10 bis 12, wobei das Werkzeug einen Haltegriff hat, der an derjenigen Seite der ersten Anlagefläche (110; 212) angebracht ist, die von der zweiten Anlagefläche (114, 214) weg weist und/oder wobei die Markervorrichtung (120; 220) sich weg von derjenigen Seite der ersten Anlagefläche (114; 214) erstreckt, die von der zweiten Anlagefläche (110; 212) abgewandt ist.

14. System nach einem der Ansprüche 10 bis 13, wobei das Werkzeug einen Drucksensor (170) aufweist, der detektiert, ob in vorbestimmten Teilbereichen der ersten und zweiten Anlageflächen (110, 114; 212, 214), die auf den einander zugewandten Seiten der ersten und zweiten Anlagefläche (110, 114; 212, 214) liegen, ein Druck ausgeübt wird.

## Claims

1. A data processing method for determining the variable position of a tool (100) comprising a first and a second level abutment area (110, 114; 212, 214) relative to a body structure (10), wherein the first abutment area (110, 114) abuts a first side of the body structure (10) and the second abutment area (212, 214) abuts a second side of the body structure (10) and the first and second abutment areas (110, 114; 212, 214) have a predetermined position relative to each other, wherein the tool comprises a tool marker device (120; 220) which has a stationary position relative to the first and second abutment areas (110, 114; 212, 214), and wherein the data processing method is performed by a computer,
wherein the following data are provided:
- tool marker position data which describe the position of the tool marker device (120; 220); and body structure marker position data which describe the position of a body structure marker device (11) which is attached to the body structure (10);
- shape data which describe the position of a shape representative of the body structure (10);
**characterised in that** the following data processing steps are performed:
- determining whether the position of the shape representative fulfils a position criterion, wherein the position criterion is that the first and/or second abutment area (110, 114; 212, 214) assumes a particular orientation relative to the shape representative;
- determining the tool marker position data and the body structure marker position data which obtain when the position criterion is determined to be fulfilled.

2. The data processing method according to claim 1, wherein the relative position between the shape representative and the first and/or second abutment area is determined on the basis of the determined tool marker position data and the determined body structure marker position data and on the basis of the shape data and tool data which describe the relative position of the first and/or second abutment area relative to the tool marker device, and/or the relative position between at least a part of the body structure and the first and/or second abutment area is determined on the basis of the determined tool marker position data, the determined body structure marker position data, the tool data and the body structure data which describe the relative position of the body structure marker device relative to at least the part of the body structure.

3. The data processing method according to claim 1 or 2, wherein: tool data are provided which describe the relative position of the first and second abutment areas of the tool relative to the tool marker device; landmark data are provided which describe the position of a first landmark in the reference system; the shape representative is an axis; the body structure is a bone; and a variable which describes the relative position between the first landmark and at least one of the abutment areas is calculated on the basis of the determined tool marker position data and the determined body structure marker position data and on the basis of the tool data, wherein the variable is a transverse distance between the first landmark and the second abutment area which runs transverse to the axis.

4. The data processing method according to claim 3, wherein the position of the intersection point between the axis and the first abutment area is calculated, wherein a line which is referred to as the joint line is calculated by dropping a first perpendicular from the calculated intersection point onto the second abutment area.

5. The data processing method according to claim 2 or 3, wherein a second perpendicular is dropped from the first landmark onto the second abutment area in order to determine the transverse distance, and/or wherein a distance between the second perpendicular and the first perpendicular or the intersection point, which is referred to as the longitudinal distance and runs along the axis, is calculated.

6. The data processing method according to any one of claims 2 to 5, wherein contact data are also provided to the data processing method which indicate whether the abutment areas are in contact with the bone, and the transverse distance and/or the longitudinal distance is calculated if the contact is indicated by the contact data.

7. The data processing method according to any one of claims 2 to 6, wherein first guidance information is generated which indicates the calculated transverse distance and/or longitudinal distance and/or wherein second guidance information is outputted which indicates the orientation of the second abutment area relative to the axis.

8. A program which, when it is running on a computer or is loaded onto a computer, causes the computer to perform the data processing method according to any one of claims 2 to 7.

9. A navigation system comprising a computer in which the program according to claim 8 is loaded or is running on the computer.

10. A system comprising the navigation system according to claim 9 and a tool (100) for detecting planes which are defined by anatomical landmarks,
comprising a first and a second level abutment area (110, 114; 212, 214) for placing onto a body structure (10),
wherein the first and second abutment areas (110, 114; 212, 214) have a predetermined position relative to each other, and
comprising a tool marker device (120; 220) which has a stationary position relative to the first and second abutment areas (110, 114; 212, 214).

11. The system according to claim 10, wherein the tool has an L-shaped form, wherein the first and second abutment areas (110, 114; 212, 214) form the L.

12. The system according to claim 10 or 11, wherein the second abutment area (214) comprises a cavity which begins between two front-facing ends (214a, 214b) of the second abutment area which face away from the first abutment area (212) and extends towards the first abutment area (212) over a majority of the length of the second abutment area (214) in the longitudinal direction.

13. The system according to any one of claims 10 to 12, wherein the tool has a holding grip which is attached to the side of the first abutment area (110; 212) which points away from the second abutment area (114; 214) and/or wherein the marker device (120; 220) extends away from the side of the first abutment area (110; 212) which faces away from the second abutment area (114; 214).

14. The system according to any one of claims 10 to 13, wherein the tool comprises a pressure sensor (170) which detects whether a pressure is being exerted in predetermined partial regions of the first and second abutment areas (110, 114; 212, 214) which lie on the mutually facing sides of the first and second abutment areas (110, 114; 212, 214).

## Revendications

1. Procédé de traitement de données pour déterminer la position variable d'un outil (100) comportant des première et seconde surfaces de butée plates (110, 114 ; 212, 214) par rapport à une structure corporelle (10), dans lequel la première surface de butée (110, 114) vient en butée contre un premier côté de la structure corporelle (10) et la seconde surface de butée (212, 214) vient en butée contre un second côté de la structure corporelle (10) et les première et seconde surfaces de butée (110, 114 ; 212, 214) ont une position relative prédéterminée l'une par rapport à l'autre, dans lequel l'outil comporte un dispositif de marqueur d'outil (120 ; 220) qui a une position fixe par rapport aux première et seconde surfaces de butée (110, 114 ; 212, 214) et dans lequel le procédé de traitement de données est mis en oeuvre par un ordinateur,
dans lequel les données suivantes sont fournies :
- données de position de marqueur d'outil qui décrivent la position du dispositif de marqueur d'outil (120 ; 220) ; et données de position de marqueur de structure corporelle qui décrivent la position d'un dispositif de marqueur de structure corporelle (11) fixé sur la structure corporelle (10),
- données de forme qui décrivent la position d'un représentant de forme de la structure corporelle (10),
**caractérisé en ce que** les étapes de traitement de données suivantes sont exécutées :
- déterminer si la position du représentant de forme satisfait à un critère de position, le critère de position étant tel que la première et/ou seconde surface de butée (110, 114 ; 212, 214) prend une orientation déterminée par rapport au représentant de forme,
- déterminer les données de position de marqueur d'outil et les données de position de marqueur de structure corporelle qui sont obtenues lorsque le critère de position est déterminé comme étant satisfait.

2. Procédé de traitement de données selon la revendication 1, dans lequel, sur la base des données de position de marqueur d'outil déterminées et des données de position de marqueur de structure corporelle déterminées ainsi que des données de forme et des données d'outil qui décrivent la position relative des première et/ou seconde surfaces de butée par rapport au dispositif de marqueur d'outil, la position relative entre le représentant de forme et la première et/ou seconde surface de butée est déterminée, et/ou sur la base des données de position de marqueur d'outil déterminées, des données de position de marqueur de structure corporelle déterminées, des données d'outil et des données de structure corporelle qui décrivent la position relative du dispositif de marqueur de structure corporelle par rapport à au moins une partie de la structure corporelle, la position relative entre au moins la partie de la structure corporelle et la première et/ou seconde surface de butée est déterminée.

3. Procédé de traitement de données selon la revendication 1 ou 2, dans lequel des données d'outil sont fournies, lesquelles données décrivent la position relative des première et seconde surfaces de butée de l'outil par rapport au dispositif de marqueur d'outil, des données de point de repère sont fournies, lesquelles données décrivent la position d'un premier point de repère dans le système de référence, et le représentant de forme est un axe et la structure corporelle est un os et, sur la base des données de position de marqueur d'outil déterminées et des données de position de marqueur de structure corporelle déterminées ainsi que des données d'outil, une grandeur décrivant la position relative entre le premier point de repère et au moins une des surfaces de butée est calculée, la grandeur étant une distance transversale s'étendant transversalement par rapport à l'axe entre le première point de repère et la seconde surface de butée.

4. Procédé de traitement de données selon la revendication 3, dans lequel la position du point d'intersection de l'axe avec la première surface de butée est calculée, dans lequel une ligne appelée ligne d'articulation est calculée, une première perpendiculaire étant tracée à partir du point d'intersection calculé sur la seconde surface de butée.

5. Procédé de traitement de données selon la revendication 2 ou 3, dans lequel pour déterminer la distance transversale, une seconde verticale est tracée à partir du premier point de repère sur la seconde surface de butée, et/ou dans lequel une distance appelée distance longitudinale, laquelle s'étend longitudinalement à l'axe, est calculée entre la seconde verticale et la première verticale ou le point d'intersection.

6. Procédé de traitement de données selon l'une quelconque des revendications 2 à 5, dans lequel d'autres données de contact sont fournies au procédé de traitement de données, lesquelles données indiquent si les surfaces de butée sont en contact avec l'os et le calcul de la distance transversale et/ou de la distance longitudinale est effectué, au cas où le contact est indiqué par les données de contact.

7. Procédé de traitement de données selon l'une quelconque des revendications 2 à 6, dans lequel sont générées des premières informations d'aide qui indiquent la distance transversale et/ou distance longitudinale calculée, et/ou dans lequel sont générées des secondes informations d'aide qui donnent une indication sur l'orientation de la seconde surface de butée par rapport à l'axe.

8. Programme qui, lorsqu'il s'exécute sur un ordinateur ou lorsqu'il est chargé dans un ordinateur, amène l'ordinateur à mettre en oeuvre le procédé de traitement de données selon l'une quelconque des revendications 2 à 7.

9. Système de navigation comportant un ordinateur dans lequel le programme selon la revendication 8 est chargé ou s'exécute sur l'ordinateur.

10. Système comportant le système de navigation selon la revendication 9 et un outil (100) pour détecter des plans définis par des points de repère anatomiques
comportant une première et une seconde surface de butée (110, 114 ; 212, 114) pour venir en butée contre une structure corporelle (10),
dans lequel les première et seconde surfaces de butée (110, 114 ; 212, 214) ont une position relative prédéterminée l'une par rapport à l'autre, et
comportant un dispositif de marqueur d'outil (120 ; 220) qui a une position fixe par rapport aux première et seconde surfaces de butée (110, 114 ; 212, 214).

11. Système selon la revendication 10, dans lequel l'outil a une forme de L, dans lequel les première et seconde surfaces de butée (110, 114 ; 212, 214) forment le L.

12. Système selon la revendication 10 ou 11, dans lequel la seconde surface de butée (214) comporte un évidement qui débute entre deux extrémités frontales (214a, 214b) de la seconde surface de butée, lesquelles extrémités sont opposées à la première surface de butée (212), et s'étend en direction de la première surface de butée (212) sur une grande partie de la longueur de la seconde surface de butée (214) dans une direction longitudinale.

13. Système selon l'une quelconque des revendications 10 à 12, dans lequel l'outil a une poignée qui est montée sur chaque côté de la première surface de butée (110 ; 212), laquelle première surface est espacée de la seconde surface de butée (114 ; 214) et/ou dans lequel le dispositif de marqueur (120 ; 220) s'étend en s'éloignant de chaque côté de la première surface de butée (114 ; 214), laquelle première surface est opposée à la seconde surface de butée (110 ; 212).

14. Système selon l'une quelconque des revendications 10 à 13, dans lequel l'outil comporte un capteur de pression (170) qui détecte si une pression est exercée dans des zones partielles prédéterminées des première et seconde surfaces de butée (110, 114 ; 212, 214), lesquelles zones se situent sur les côtés opposés l'un à l'autre des première et seconde surfaces de butée (110, 114 ; 212, 214).
